# EUROPEAN PATENT APPLICATION

(11) **EP 4 494 554 A1**
(43) Date of publication of application: **22.01.2025**
(21) Application number: 24184475.2
(22) Date of filing: 25.06.2024
(51) Int. Cl.: A61B 5/05, A61B 5/00, G01N 27/00

(54) **ELECTRIC FIELD GENERATION**

(30) Priority: 19.07.2023 GB 202311057
(71) Applicant: HM Technology International Limited, Harrow HA1 2EY (GB)
(72) Inventor: Mamigonians, Hrand, London, W5 3EB (GB)
(74) Representative: Atkinson & Company Intellectual Property Limited

(57) **Abstract**

Penetrating matter (non-organic, organic or biological) with electric fields is shown, to identify one or more properties of the matter. A first electrode **(111)** is energized and an output signal from a capacitively coupled second electrode **(115)** is monitored. The output signal is processed to identify properties of the matter (which for biological matter could indicate the presence of cancer). The first electrode and the second electrode are selected from a plurality of electrodes mounted on a first planer surface of a dielectric substrate **(401).** The electrodes are circumferentially and substantially evenly displaced upon the planer surface around a hole in the substrate.

## Description

The present invention relates to an apparatus for generating electric fields, wherein the electric fields are suitable for penetrating matter to identify one or more properties of the matter.

The present invention also relates to a method of penetrating matter with electric fields to identify one or more properties of the matter.

A sensor array for sensing the electrical permittivity of an object is disclosed in GB2488623. A dielectric layer presents a surface defining the base of a volume in which a test object is placed. An electrically active layer beneath the dielectric layer is also provided. A first set of electrodes extend in a first direction and a second set of electrodes extend in a second direction that is perpendicular to the first, such that each one of the first set of electrodes intersects with one of the second set of electrodes. An input signal is applied to a first electrode or the first set of electrodes, thereby generating an electric field that extends outside the sensor array and into the volume. Output signals are detected in each one of the second set of electrodes that intersects the first electrode. The output signals are caused by capacitive coupling between the first electrodes and each one of the electrodes in the second set of electrodes, and are indicative of the electrical permittivity of the volume above the intersection of the first electrode and each one of the electrodes of the second set of electrodes.

An array of this type has been considered for detecting properties of organic material and, in particular, human skin to detect skin cancers. GB2577927 describes an application of this type, in which probe calibration may be achieved by scanning a reference region of skin that does not include the skin condition and then deploying the probe against the skin condition to produce test data.

In an environment where medical decisions may be based on an analysis of biological matter, an accurate and reliable testing procedure is required. In this respect, a first problem exists in that when many electrodes are present, of which many are not in actual use for a scanning operation, these electrodes become charged and as such these charges may disrupt the electric fields required for the scanning operation.

According to a first aspect of the present invention, there is provided an apparatus for generating electric fields, wherein said electric fields are suitable for penetrating matter to identify one or more properties of said matter, comprising: a first dielectric substrate presenting a first planar surface and a second planar surface, with a hole having a first radius; a plurality of circumferentially and substantially evenly displaced scanning electrodes on said first planar surface, located at a second radius around said hole; and respective electrical conductors from each said scanning electrode configured to pass through said first dielectric substrate to said second planar surface.

In an embodiment, the apparatus comprises a plurality of circumferentially and substantially evenly displaced secondary electrodes on said first planar surface, wherein: said secondary electrodes are positioned at a third radius; and said third radius is larger than said second radius. Each said secondary electrode is radially aligned with a respective scanning electrode. The dielectric substrate is implemented as a first circuit board; said first circuit board is substantially circular and has a fourth radius that is larger than said third radius.

According to a second aspect of the invention, there is provided a method of generating electric fields for penetrating matter to identify one or more properties of said matter, comprising the steps of: locating an apparatus such that a hole of a first radius in a dielectric substrate is at the position of said matter; and energizing the apparatus to produce said electric fields, wherein: said dielectric substrate presents a first planar surface and a second planar surface; a plurality of circumferentially and substantially evenly displaced scanning electrodes are located on said first planar surface at a second radius around said hole; and respective electrical conductors from each said scanning electrode are configured to pass through said first dielectric substrate to said second planar surface.

Embodiments of the invention will be described, by way of example only, with reference to the accompanying drawings. The detailed embodiments show the best mode known to the inventor and provide support for the invention as claimed. However, they are only exemplary and should not be used to interpret or limit the scope of the claims. Their purpose is to provide a teaching to those skilled in the art. Components and processes distinguished by ordinal phrases such as "first" and "second" do not necessarily define an order or ranking of any sort. In the drawings:
*Figure 1* shows an apparatus for generating electric fields;
*Figure 2* shows the introduction of substantially evenly displaced secondary electrodes;
*Figure 3* shows the introduction of a circular hole;
*Figure 4* shows an implementation of the dielectric substrate as a circular circuit board;
*Figure 5* shows the introduction of a second circuit board that is parallel with the first circuit board introduced in *Figure 4*;
*Figure 6* shows the first circuit board and a schematic representation of the electronics contained on the second circuit board;
*Figure 7* shows the apparatus located within a housing to facilitate application upon a subject's skin;
*Figure 8* shows an arrangement for examining specimens;
*Figure 9* shows an alternative arrangement for examining specimens;
*Figure 10* shows the arrangement of *Figure 9* with an additional dielectric material;
*Figure 11* shows procedures performed by the processor identified in *Figure 6**;*
*Figure 12* shows an example of a scanning cycle;
*Figure 13* details procedures for energizing electrodes identified in *Figure 12**;*
*Figure 14* shows the production of output samples;
*Figure 15* details procedures for identifying and storing information identified in *Figure 13*; and
*Figure 16* shows a data table.

### Figure 1

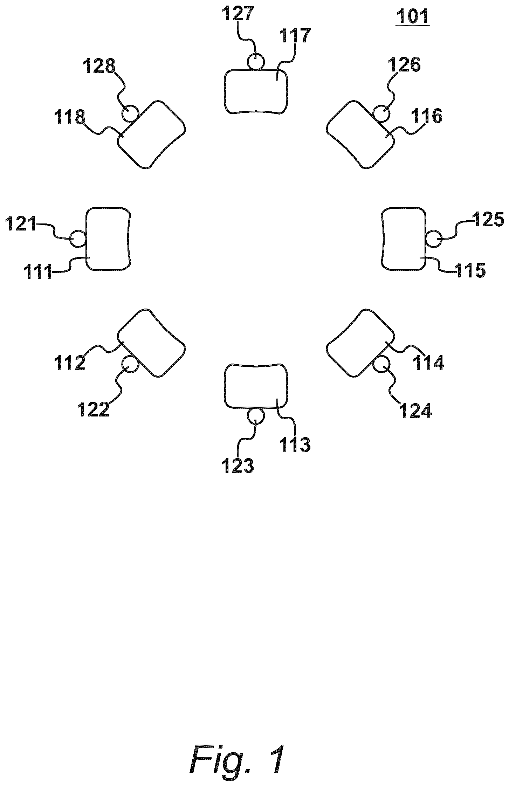

An apparatus for generating electric fields is shown in *Figure 1* that is suitable for penetrating matter to identify one or more properties of the matter. The matter could be inorganic, organic or biological. The biological matter could comprise human skin and a test could be performed upon a person to identify cancerous skin for example. Alternatively, the biological material could consist of a specimen removed from a subject for analysis.

A dielectric substrate presents a first planar surface **101** as shown in *Figure 1**.* Circumferentially and substantially evenly displaced scanning electrodes are attached to the first planar surface **101.** In the example shown *in* *Figure 1*, eight scanning electrodes are provided, consisting of a first scanning electrode **111**, a second scanning electrode **112**, a third scanning electrode **113**, a fourth scanning electrode **114**, a fifth scanning electrode **115**, a sixth scanning electrode **116**, a seventh scanning electrode **117** and an eighth scanning electrode **118.** In alternative configurations, fewer electrodes may be provided (possibly a total of four) or more electrodes may be provided, such as sixteen.

Each scanning electrode includes an electrical conductor, comprising a first conductor **121** to an eighth conductor **128**, attached to the first scanning electrode **111** to the eighth scanning electrode **118** respectively, which pass through the dielectric substrate to a second a planar surface on the opposite side of the substrate. In this way, it is possible for the scanning electrodes **111** to **128** to be energized or monitored, while minimising the presence of additional conductors on the first planar surface, which could become polarized and thereby introduce errors.

### Figure 2

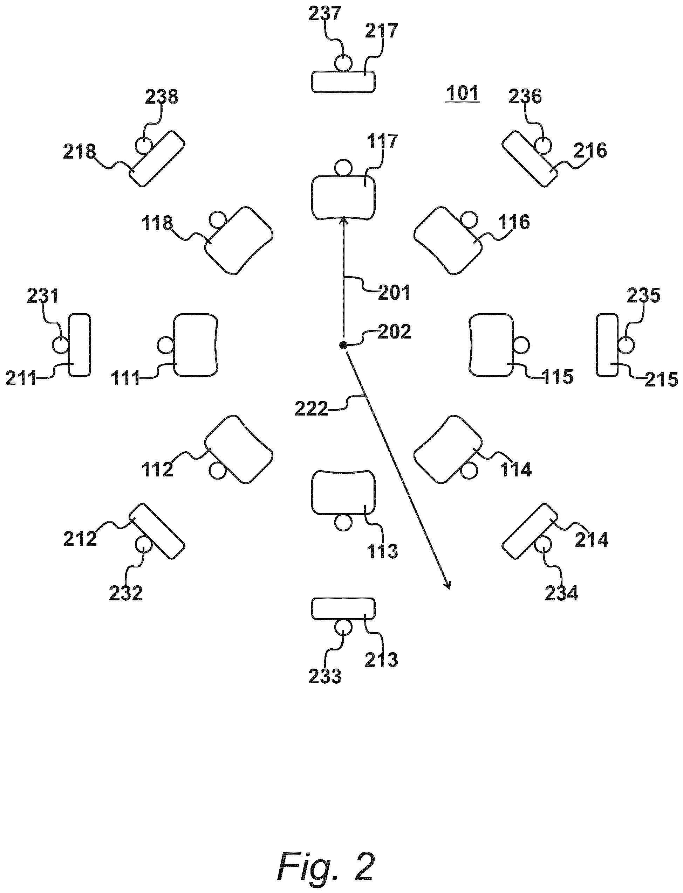

In an embodiment, as illustrated in *Figure 2*, circumferentially and substantially evenly displaced secondary electrodes are also introduced to the first planar surface **101.** The scanning electrodes **111** to **118** are positioned at a first radius **201** from a central point **202.** The secondary electrodes comprise a first secondary electrode **211**, a second secondary electrode **212**, a third secondary electrode **213**, a fourth secondary electrode **214**, a fifth secondary electrode **215**, a sixth secondary electrode **216**, a seventh secondary electrode **217** and an eighth secondary electrode **218.** The secondary electrodes are positioned at a second radius **222** from the central point **202** and the second radius **222** is larger than the first radius **201.**

In the embodiment of *Figure 2*, each secondary electrode (electrode **211** for example) is radially aligned with a respective scanning electrode (**111**).

Each secondary electrode **211** to **218** also includes a secondary electrical conductor, identified as secondary electrical conductors **231** to **238** respectively. These pass through the dielectric substrate to the second planar surface on the opposite side of the substrate.

### Figure 3

In accordance with an aspect of the invention, a circular hole **301** is cut in the first dielectric substrate, which has a third radius **302** from the central point **202**, that is smaller than the first radius **201.**

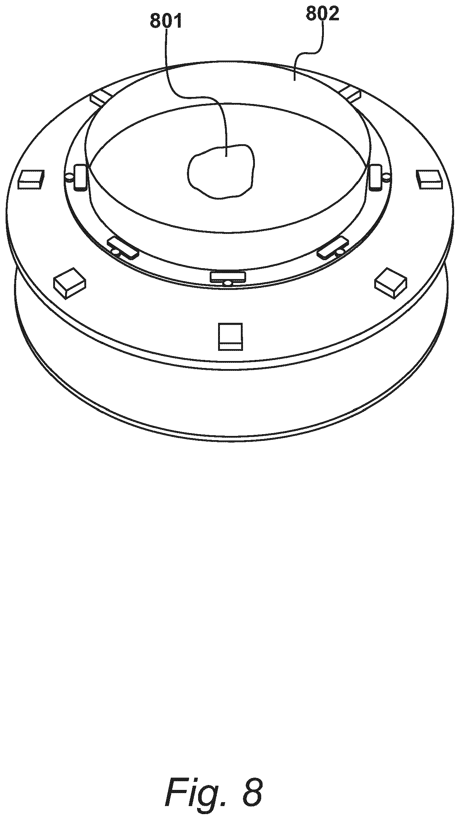

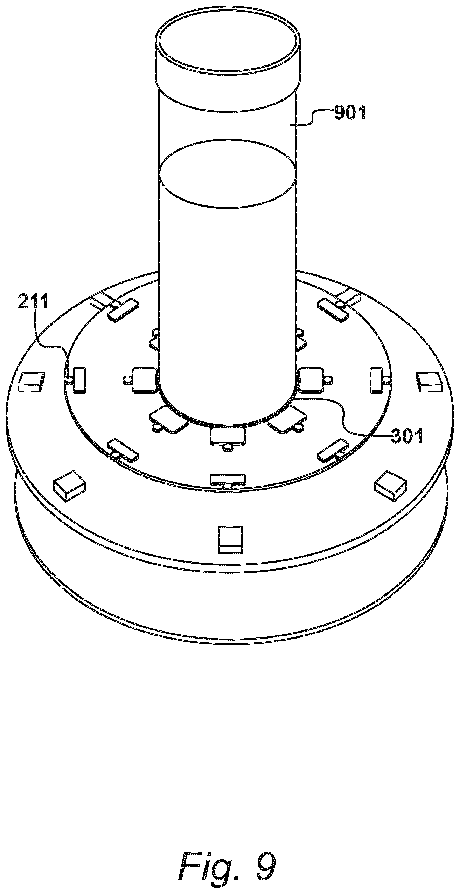

Electric fields radiate away from this hole **301** in three-dimensional space. When considering a skin condition, a housing containing the apparatus may be located upon a subject, such that the skin condition itself occupies the region where the hole is present. As such, no physical electrode contact is made upon the actual skin condition being investigated, during scanning operations in which electric fields penetrate the skin condition. Where contact is made with the skin, around the skin condition of interest, the electrodes are electrically insulated. In an embodiment, this insulation is provided by a glass cover. Over the position of the circular hole, the glass cover is transparent to facilitate optical viewing of the skin condition by a clinician. Around the hole, at the position of the electrodes, the glass cover may be opaque. The hole may also be used to receive a receptacle, such as a tube, as described with reference to *Figure 8* and *Figure 9**.* The receptacle may be of a standard type used for storing specimens in formalin (methanal in aqueous solution) and examination may take place with the formalin present.

### Figure 4

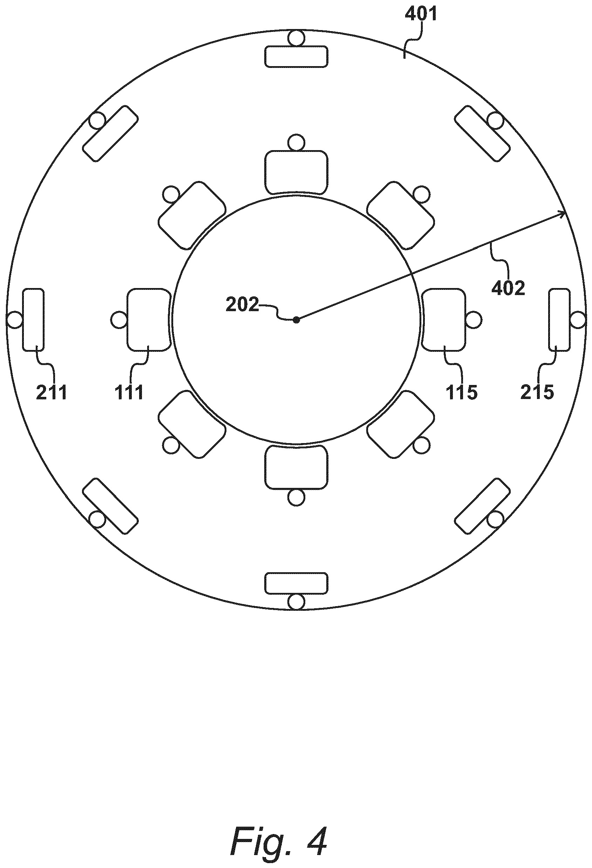

In an embodiment, the dielectric substrate **101** is implemented as a first circuit board **401** which is circular and has a fourth radius **402** from the central point **202** that is larger than the second radius **222.** Thus, in this embodiment, the electrical conductors (**121 - 128**) from the primary electrodes, and similar electrical conductors (**231 - 238**) from the secondary electrodes, pass through the circuit board to present electrical connections on the opposite side of the circuit board to that shown in *Figure 4**.*

### Figure 5

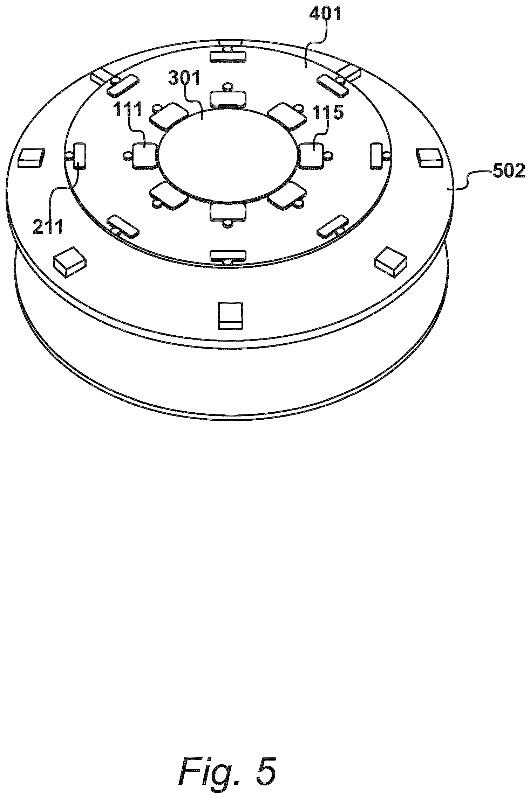

The first circuit board **401** is also shown in *Figure 5**.* In addition, there is provided a second circuit board **502** that is parallel with the first circuit board **401.** In this embodiment, the second circuit board is also circular and has a radius that is greater than the radius **402** of the first circuit board. In an alternative embodiment, the second circuit board is of a similar dimension and this approach may be beneficial when reducing overall size for a hand-held unit.

The second circuit board **502** includes electronics for energizing and monitoring the scanning electrodes (**111 - 118**), and for changing the properties of the secondary electrodes (**211 - 218**). The electronics also includes data storage devices for the storage of data and executable instructions controlling the operation of the device, In an embodiment, executable instructions may be updated from external sources and as such may be identified a firmware.

### Figure 6

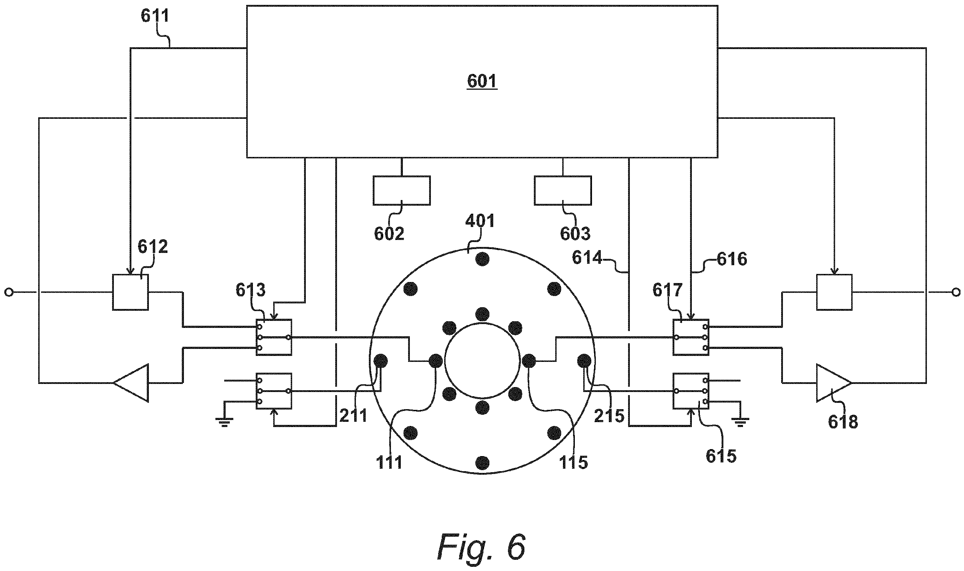

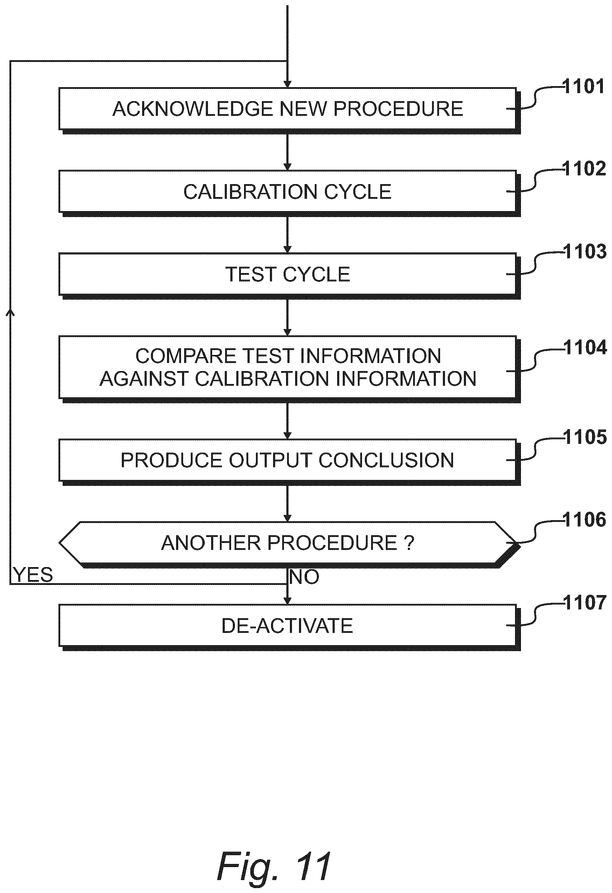

A schematic representation of the first circuit board **401** is shown in *Figure 6**,* along with a representation of the electronics contained on the second circuit board **502.** The electronics includes a processor **601** and operations performed by the processor **601** will be described with reference to *Figure 11* to *Figure 16**.*

An embodiment includes light-emitting devices **602** for illuminating the apparatus and a lens, thereby enhancing a clinician's ability to view the area under examination. These components are particularly useful when examining skin and allow the clinician to compare electrical characteristics (permittivity and conductivity) against visual images. In an embodiment, the conductivity and permittivity data derived from the scanning operations may be displayed to a clinician on a suitable display device such as a laptop computer.

*Figure 6* shows electronics relevant to the first scanning electrode **111**, the first secondary electrode **211**, the fifth scanning electrode **115** and the fifth secondary electrode **215.** However, it should be appreciated that this electronics is repeated, such that all of the scanning electrodes and all of the secondary electrodes are connected in a similar way.

The electronics shown in *Figure 6* allows the first scanning electrode **111** to be energized while the fifth scanning electrode **115** is monitored. Alternatively, it also provides for the fifth scanning electrode **115** to be energized while the first scanning electrode **111** is monitored. In addition, both the first secondary electrode **211** and the fifth secondary electrode **215** may be connected to ground or may be allowed to float (open circuit). Furthermore, in an alternative embodiment, a connection to ground may be made via a variable resistor, such that intermediate resistivity values may be established and additional intermediate data values may be obtained. In an embodiment, all of the non-selected scanning electrodes float; that is to say, they are not connected to ground as this will tend to attract the electric field away from the monitoring scanning electrode. Thus, in this way, the electric field is focussed towards the monitoring electrode.

For the purposes of this description, it will be assumed that the first electrode **211** is being energized while the fifth electrode **115** is being monitored. In this embodiment, two energizations take place: for the first, the fifth secondary electrode is grounded and for the second the fifth secondary electrode floats. The first secondary electrode is not adjusted for this operation and is allowed to float. In an alternative embodiment, more than two energizations would take place.

The processor **601** provides an energizing control signal on a first control line **611** to a first switch **612.** The switch receives as its input a constant scanning voltage which is in turn supplied to a second switch **613.** When activated, the first switch **612** sends an energizing strobing pulse to the first scanning electrode **111** via the second switch **613.** The first scanning electrode **111** is capacitively coupled to the fifth scanning electrode **115** that is being monitored. The electric field enters matter placed on the first circuit board **401** along many paths that follow a three-dimensional trajectory having components that are perpendicular to the plane of *Figure 6**.* The duration of the analogue strobing pulse is determined by the first control signal under the control of the processor **601.**

A second control line **614** activates a third switch **615** that, on a first energizing operation, connects the fifth secondary electrode **215** to ground. As a result of this, the electric field is also attracted to this grounded electrode; thereby changing the distribution of the electric field in three-dimensional space. Furthermore, a third control line **616** operates a fourth switch **617**, such that the monitored signal is supplied to the processor **601** via an amplifier **618.**

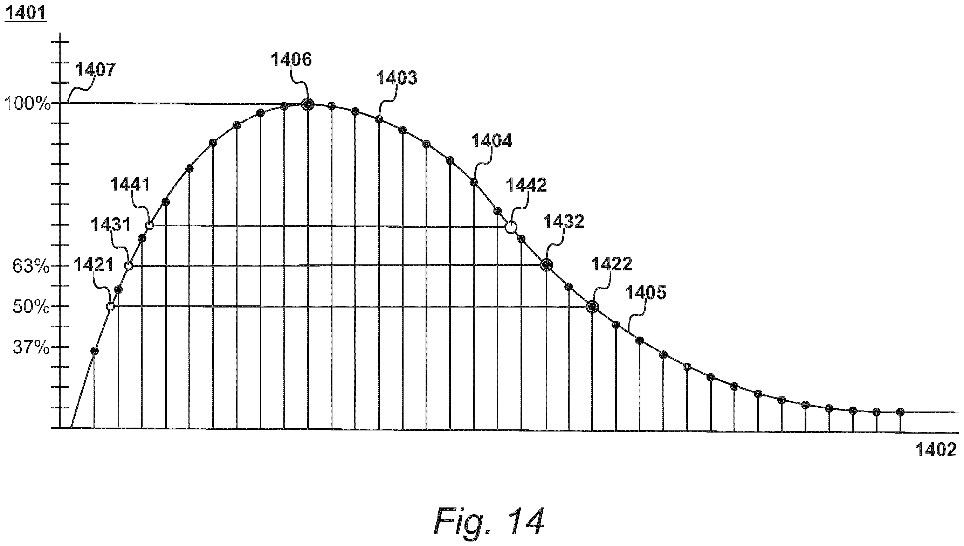

An analogue output from the amplifier **618** is sampled, as described with reference to *Figure 14*, and a further scanning operation is then performed, after operation of the third switch **615**; such that the fifth secondary electrode **215** is allowed to float. During this second scanning operation, the electric field is less attracted to the fifth secondary electrode, which results in a different distribution of the electric field within three-dimensional space.

These two scanning operations, using the same two capacitively connected scanning electrodes, obtain different results; particularly given that differing levels of penetration will occur. Thus, by considering these two different levels of penetration, it is possible to determine the extent of changing characteristics of the matter under examination at different depths. This can be particularly useful when considering skin cancer for example, given that if an anomaly is present, an indication can be derived as to the extent of its depth and as such provide an indication of greater or lesser threat.

In an embodiment, a significant amount of data is collected, given that it is possible for each of the scanning electrodes (of which there are eight in the embodiment of *Figure 6*) to be selected as an energized electrode. Furthermore, for each energized electrode, all of the remaining (seven in this embodiment) electrodes may be considered as a monitored electrode. Thus, a total of fifty-six combinations are possible (for this particular embodiment) and a scanning operation is performed twice for each combination, thereby giving a total of one hundred and twelve datasets for each cycle.

### Figure 7

The presence of circular hole **301** makes the apparatus described with reference to *Figure 5* particularly suitable for examining skin conditions. A suspicious skin condition may be identified that requires further tests to determine whether further examination and treatment are required.

When using electric-field-generating electrodes, it is undesirable for an airgap to exist between the skin and the electrodes. However, if the electrodes are brought into contact with the skin condition itself, this can change characteristics of the skin and thereby produce erroneous results. Thus, the configuration described with reference to *Figure 5* allows the electrodes to be brought into contact with the skin, thereby avoiding the introduction of an airgap, but not in contact with the actual skin condition being examined.

It is not necessary, nor desirable, for the conducting electrodes to be in direct contact with the skin. In an embodiment, a glass plate is provided that makes physical contact with the skin. This ensures that there is no airgap and maintains consistent positioning of the electrodes from the skin. The apparatus does not conduct an electrical current into the skin. The apparatus creates an electric field that penetrates the glass cover and the skin tissue.

The circular hole **301** surrounds the skin condition along with the scanning electrodes **112** to **118** and the secondary electrodes **211** to **218.** Thus, in a deployment for the examination of skin, the apparatus described with reference to *Figure 5* is effectively inverted.

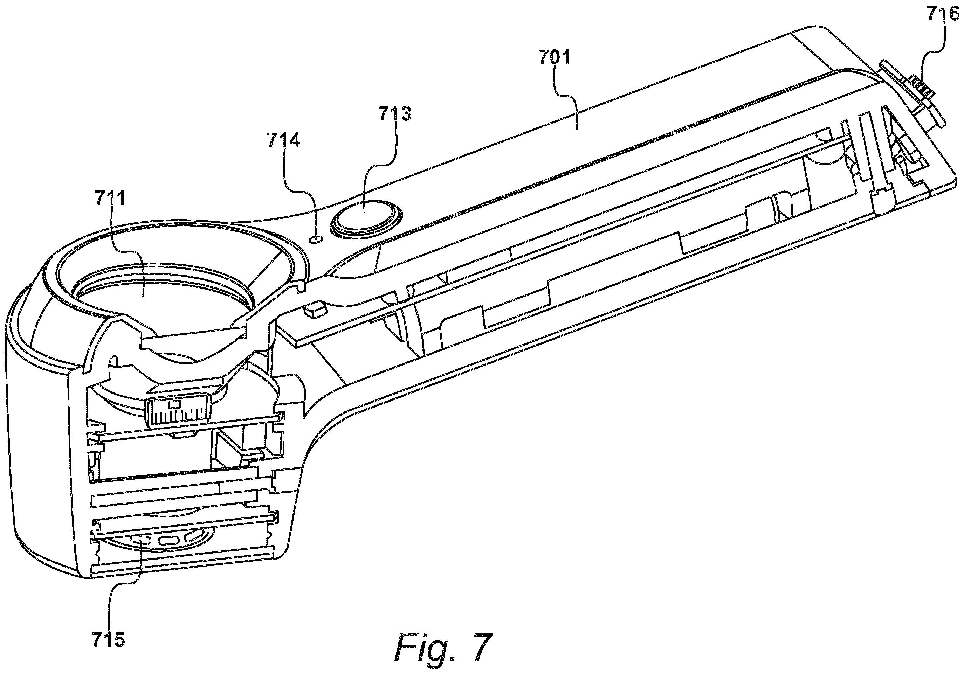

In an embodiment, for the examination of skin conditions, it is possible for the apparatus described with reference to *Figure 5* to be located within a housing **701** to facilitate application upon a subject's skin. In this embodiment, the hole **301** is extended through the apparatus allowing a clinician to make a visual examination prior to aligning the skin condition for the electric field examination. In an embodiment, the hole is capped with a lens at a viewing end, to enhance visibility, and capped by a glass plate at the contact end. The embodiment of Figure 7 includes a lens **711** to facilitate visual inspection. In an embodiment, the lens at the top and the glass plate at the bottom effectively seal the apparatus to prevent fluid entry.

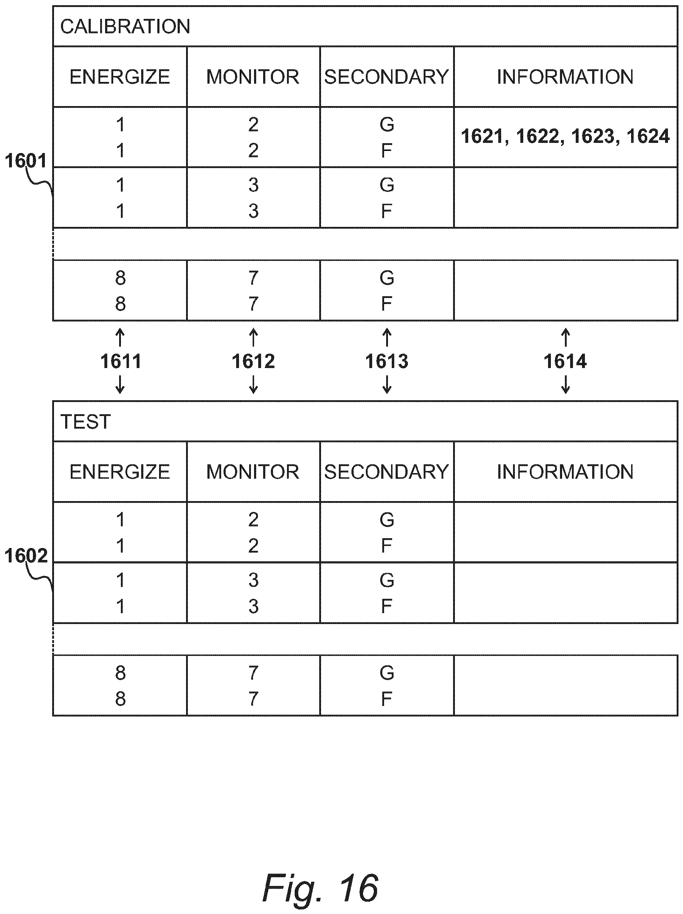

To produce meaningful results, it is necessary for the data generated while testing to be compared against calibration data. For the apparatus shown in *Figure 7*, the apparatus may firstly be located upon an area of healthy skin which does not show any evidence of a skin condition existing. In this position, a full scanning cycle is performed to produce calibration data, as described with reference to *Figure 14*, which is then processed to generate calibration information as described with reference to *Figure 16**.* Having performed the calibration cycle, the housing **701** is then brought to the position of the skin condition, such that a testing cycle is performed that generates test data and test information that are similar to the calibration data and calibration information. In alternative arrangements, the calibration data may be produced while the apparatus is open to ambient air.

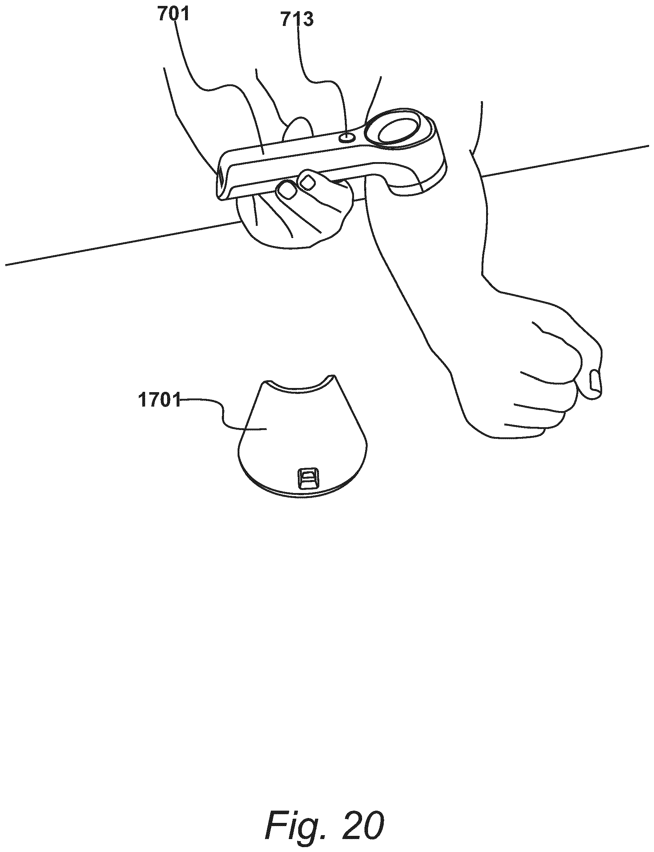

The apparatus shown in Figure 7 may communicate wirelessly with processing equipment such as a laptop computer. A first scanning cycle, to produce reference data, is initiated by applying pressure to a button **713.** In response to this, a light emitting indicator **714** emits light of a different colour to indicate that a scanning cycle is in progress. At the end of the scanning cycle, the colour of light emitted changes back to its passive colour or alternatively may not be illuminated at all. The apparatus is then deployed, as described with reference to Figure 20, to produce test data and a scanning procedure for the generation of test data is again initiated by applying pressure to button **713.**

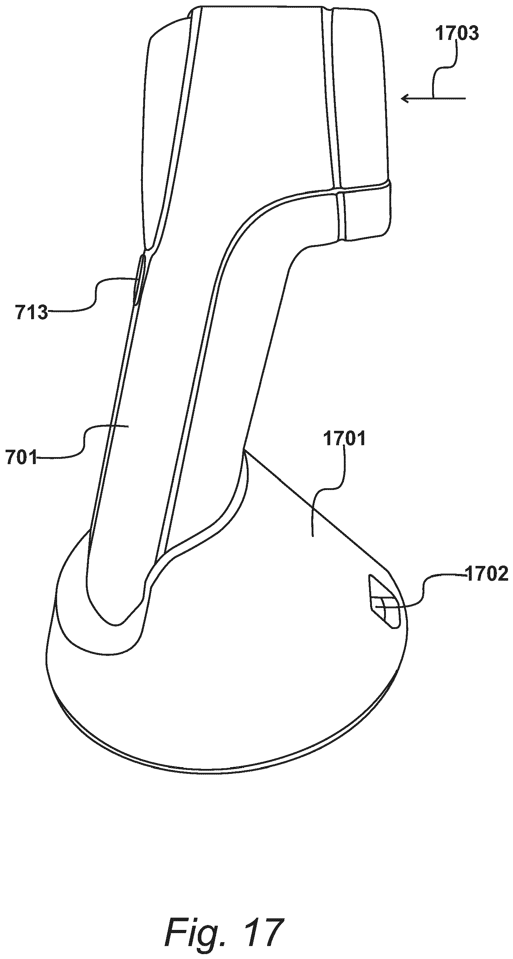

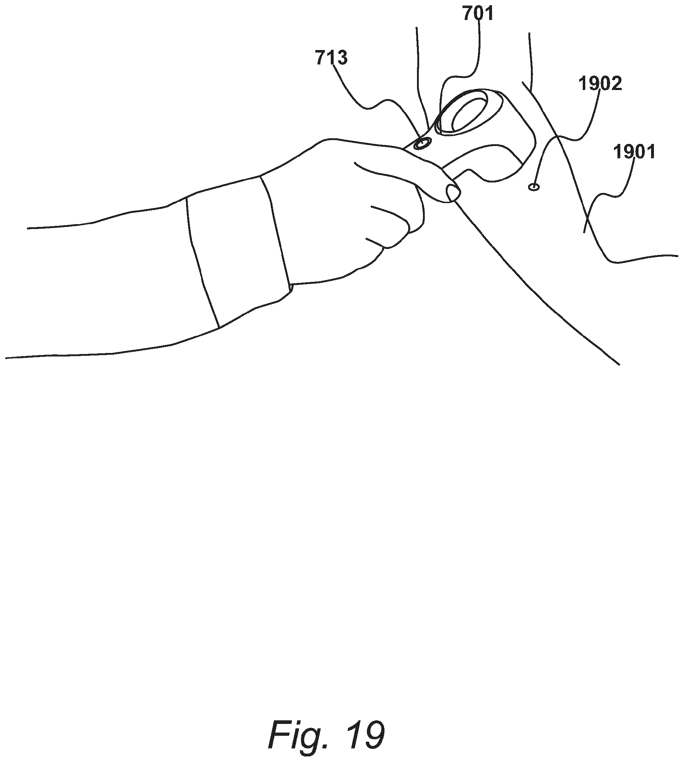

To enhance visual inspection, as described with reference to *Figure 19*, the area being inspected is illuminated by light emitting devices **715.** To conserve power, light emitting devices **715** are usually not energized. In an embodiment, the LED devices **715** are not energized when the apparatus is located within a charging cradle, as described with reference to *Figure 17**.* Upon removing the apparatus **701** from a cradle, the light emitting devices **715** are energized to facilitate visual observation and also to indicate to a user that the probe is ready to perform scanning operations.

A plug **716** extends from the bottom of the probe housing to facilitate the charging of internal batteries when the apparatus is secured within a cradle. Furthermore, as an alternative to transmitting data wirelessly, data communication may take place by storing data within the apparatus and then effecting a download of data when returned to a cradle.

Data communication with the apparatus may also be performed to perform a firmware update, either via a connected lap top computer or directly via a wireless connection.

### Figure 8

The arrangement described with reference to *Figure 5* may also be deployed for examining specimens. Thus, in a clinical situation, it is possible for a biopsy to be taken and for analysis to be performed immediately, as an alternative to, or in addition to, sending a sample for laboratory testing.

In the embodiment shown in *Figure 8**,* a specimen **801** is placed in a container **802** surround by an appropriately electrically inert liquid. As shown in *Figure 8**,* the container **108** is located upon the first planar surface **101.** The scanning electrodes **111** to **118** are energized and monitored as previously described. Alternatively, specimens can be examined as received in containers of formalin.

When examining specimens as shown in *Figure 8**,* it is also necessary to perform a calibration cycle before the actual testing of the specimen is performed. In this example, calibration data is generated by performing a complete scanning operation without the container **802** being present. Thus, the calibration data effectively represents characteristics of the ambient air. Alternatively, a similar container containing formalin but with no specimen may be used to create the reference data.

### Figure 9

As an alternative to placing a wide container upon the planar surface, as described with reference to *Figure 8*, it is possible for a tube **901** to be located within the circular hole **301.** Advantages of this approach are that the specimen itself requires less surrounding liquid and the tube is held firmly in position while a scanning operation is performed. However, compared to the arrangement described with reference to *Figure 8**,* the secondary electrodes **211** to **218** will have less influence.

### Figure 10

Experiments have shown that it is also possible to place a ring **1001** around the tube **901** to modify the concentration of electric fields. In an embodiment, a ring **1001** is made from a dielectric material such that, after a few scanning operations, the material becomes charged and as such, the presence of this charge influences the electric fields generated by the scanning electrodes.

### Figure 11

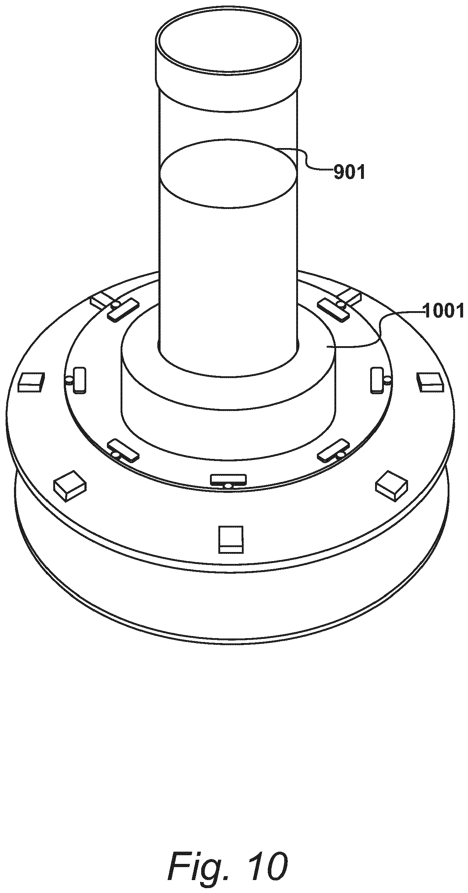

Procedures performed by the processor **601** when deploying a skin examination apparatus, as described with reference to *Figure 7*, or when deployed for a specimen analysis as described with reference to *Figure 8* to *Figure 10**,* are shown in *Figure 11**.* At step **1101**, the processor acknowledges that a new procedure is to be conducted and appropriately coloured light emitting devices may be illuminated or a message may be presented on a connected laptop computer. Prior to this, the processor **601** will have checked that information from a previous procedure has been transferred and that working memory may now be overwritten.

At step **1102**, a calibration cycle is performed and at step **1103** a test cycle is performed. These scanning procedures for both the calibration cycle and the test cycle are substantially similar and the fundamental difference relates to what is actually being examined. Thus, for the apparatus described with reference to *Figure 7*, calibration may be performed upon healthy skin, whereafter a test cycle is performed in relation to the potential skin condition.

The calibration cycle and the test cycle both generate a significant amount of data as described with reference to *Figure 14*. This data is analysed to produce a smaller volume of information as described with reference to *Figure 16*; for both the calibration cycle and the test cycle. At step **1104**, the test information is compared against the calibration information and an output conclusion is generated at step **1105.**

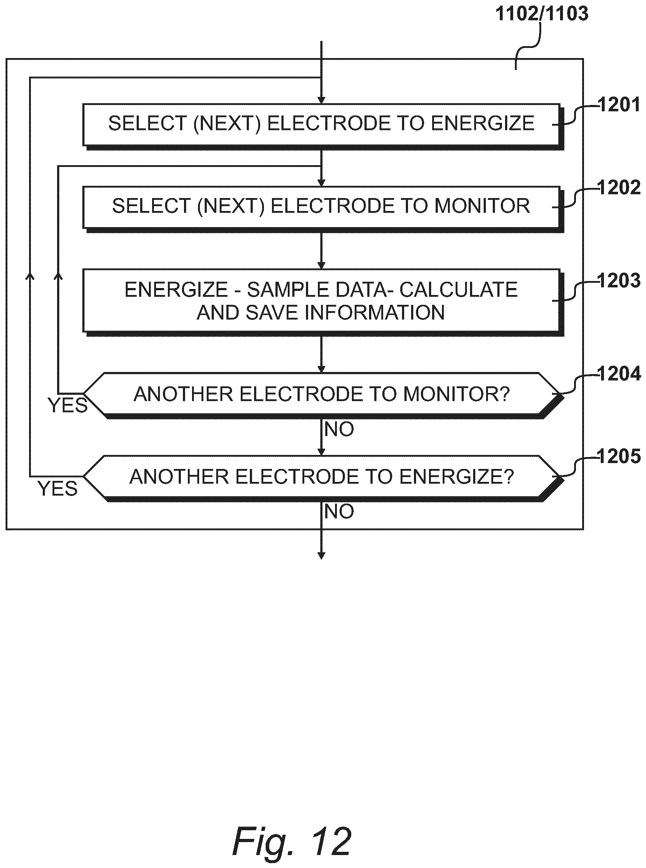

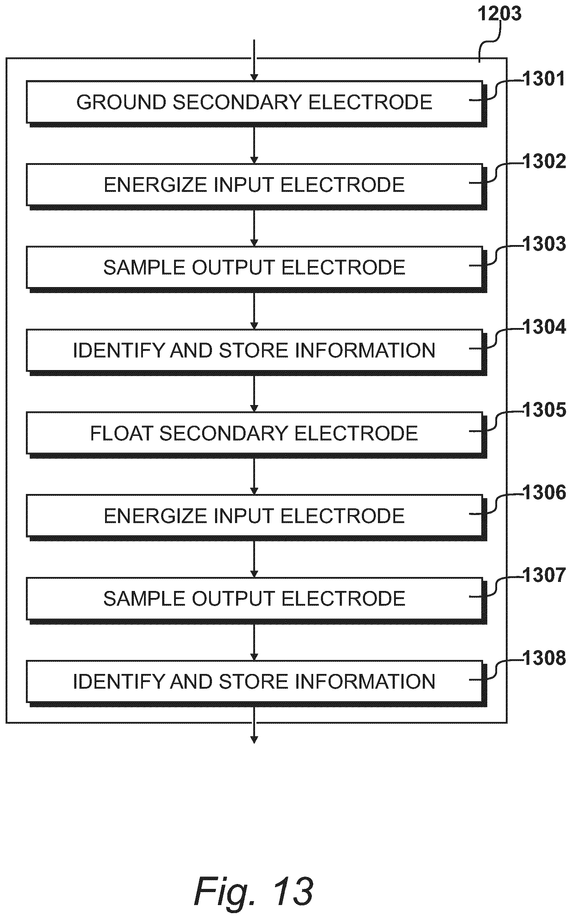

In this embodiment, it is assumed that the device is totally self-contained and is in a position to produce local output. In alternative embodiments, a graphical user interface is provided to a user by means of a laptop computer which may receive the output conclusion, partially-processed information or the raw data, such that some of the procedures described with reference to *Figure 12* and *Figure 13* may be performed on the laptop computer, as an alternative to being performed fully within processor **601.**

After producing the output conclusion at step **1105**, a question is asked as to whether a new procedure is to be performed and when answered in the affirmative, this position is again acknowledged at step **1101.** Alternatively, if the question asked at step **1106** is answered in the negative, the system is deactivated at step **1107.**

### Figure 12

An example of a scanning cycle is shown in *Figure 12*. A substantially similar cycle is performed for both the calibration cycle **1102** and the test cycle **1103.** At step **1201**, a scanning electrode is selected to be energized which, for the purposes of this example, could be the first scanning electrode **111.**

At step **1202**, an electrode is selected to be monitored which, for the purpose of this example, may be the second scanning electrode **112.**

At step **1203**, the electrode selected at step **1201** is energized and the electrode selected at step **1202** is monitored. Data is saved and calculations are performed to generate information that is also saved.

At step **1204**, a question is asked as to whether there is another electrode to monitor which, when answered in the affirmative, results in the next electrode being selected at step **1202.** Thus, in this example, the first scanning electrode **111** is energized again with the third scanning electrode **113** being monitored.

Thus, the question asked at step **1204** will continue to be answered in the affirmative until all of the remaining scanning electrodes have been selected as an electrode to be monitored. When the question asked at step **1204** is answered in the negative, a question is asked at step **1205** as to whether another electrode is present to be energized. Thus, on a first iteration, the question asked at step **1205** will be answered in the affirmative and the next electrode will be selected at step **1201.** Thus, for the purpose of this example, the second scanning electrode **112** may be selected at step **1201** with the third scanning electrode **113** being selected at step **1202.**

Again, eventually, the question asked at step **1204** will be answered in the negative and the next electrode will be selected to be monitored. As this continues, the question asked at step **1205** will be answered in the negative when all of the electrodes present have been selected at step **1201.**

The procedure described with reference to *Figure 12* ensures that every possible combination of energizing electrodes and monitoring electrodes has been considered. The sequential ordering described is attractive given that it allows simple counters to be incremented. However, fundamentally, the selection of energizing and monitoring electrodes may take place in any order. In an embodiment, non-selected scanning electrodes are not grounded. However, these will tend to attract the electric field away from the monitoring electrode. Thus, in an alternative embodiment, the non-selected scanning electrodes float to achieve improve electric field focussing.

### Figure 13

Procedures **1203** for energizing the electrodes, identified in *Figure 12*, are detailed in *Figure 13*. An electrode to be energized has been selected and an electrode to be monitored has been selected.

At step **1301**, secondary electrode **215** is grounded. At step **1302**, the input electrode **111** is energized and at step **1303** an output signal from the monitored electrode **115** is sampled. In an embodiment, each energizing pulse lasts for a duration of ten micro-seconds (10 µs) and individual pulses are separated by a duration of ninety-microseconds (90 µs). During each cycle, one hundred samples are taken, which requires a sampling rate of five megaHertz (5 MHz).

An analogue-to-digital converter, forming part of the processor **601**, converts each sample into a twelve-bit representation and as a result of this, each energizing pulse generates a significant amount of data. However, the processor **602** is fast enough to allow a significant amount of processing to take place during the sample period. Thus, by comparing samples, it is possible to identify a peak value and the regular intervals between samples allows the time at which this peak value occurred to be determined. Thus, each sample point is made up of data that defines a voltage level at a particular time.

This raw data is saved at step **1303.** The raw data is then processed to produce a smaller volume of information that is stored at step **1304.** At step **1305**, the fourth switch **617** is operated, such that the fifth secondary electrode **215** now floats.

The procedures identified above are now repeated. Thus, at step **1306**, the input electrode **111** is energized and at step **1307** an output from the monitored electrode is sampled. Information is then identified and stored at step **1308.**

### Figure 14

The production of output samples at step **1303**, or at step **1307**, is illustrated in *Figure 14**.* In the representation of *Figure 14*, output voltage **1401** is plotted against time **1402.** The sampling operation creates data points, such as data point **1403** and data point **1404.** As is well known in the art, it is also possible to fit a curve **1405** to the existing data points, such that values on this curve may be calculated to a higher degree of accuracy through a process of interpolation. From this, it is possible to identify a peak value **1406.**

Having calculated the peak value **1406,** it is then possible to identify points at which a proportion of this peak value has been presented. Thus, from the large volume of raw data, it is possible to calculate a much-limited volume of information which conveys what is required in terms of a peak and a rate of discharge. In particular, the absolute peak value and the rate of discharge are related to the conductivity and permittivity of the material.

### Figure 15

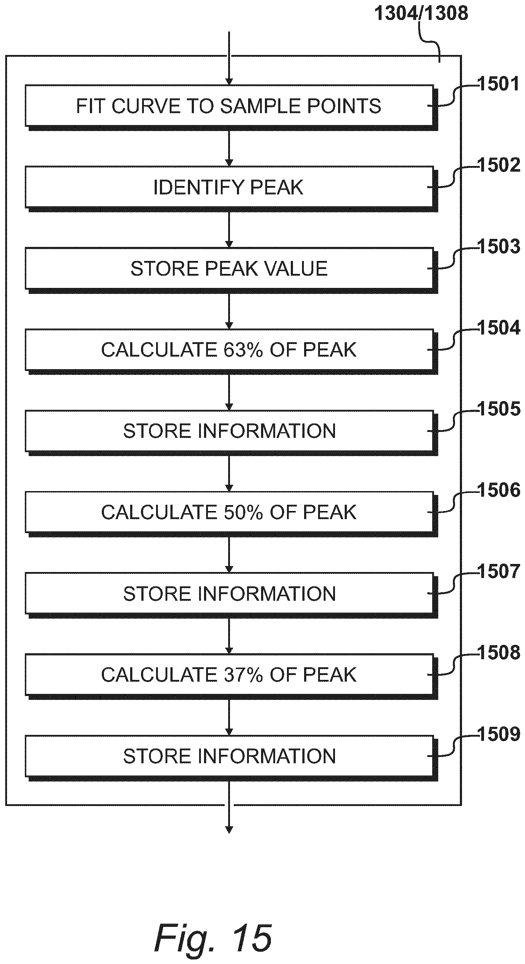

Procedures **1304** and **1308** for identifying and storing information are shown in *Figure 15**.* At step **1501**, a mathematical representation of curve **1405** is calculated in an exercise of fitting sample points **1403**, **1404** etc to a mathematically defined curve.

At step **1502**, the peak **1406** is identified and this peak information **1407** is stored at step **1503.**

At step **1504**, a value is calculated that represents sixty-three percent (63%) of the peak value **1407.**

At step **1505**, information is identified, consisting of a first data point **1421** and a second data point **1422** at which the curve **1405** passes through the sixty-three percent value.

At step **1506**, a value is calculated that represents fifty percent (50%) of the peak value **1407.** A first data point **1431** and a second data point **1432** are identified where the curve 1405 crosses this fifty percent level.

At step **1508**, a value is calculated that represents thirty-seven percent (37%) of the peak value **1407.** Again, at step **1509**, a first data point **1441** is calculated, along with a second data point **1442** showing where the curve **1405** crosses these values.

### Figure 16

The information calculated at steps **1502**, **1504**, **1506** and **1508** is stored in a database and a representation of this database is illustrated in *Figure 16**.*

A data table **1601** is constructed for the calibration data and a similar data table **1602** is constructed for the test data. In a first column **1611**, the electrode being energized is recorded and in a second column **1612** the electrode being monitored is recorded. For each of these combinations, a third column **1613** records whether the relevant secondary electrode was grounded or allowed to float. The resulting information is then stored in a fourth column **1614.**

In this embodiment, the information represents the first peak value **1621.** The information then represents the two positions for thirty-seven percent of the peak value **1622**, the two positions for fifty percent of the peak value **1623** and the two positions for sixty-three percent of the peak value **1624.**

After fully populating the database table of *Figure 16*, the resulting information consists of a relatively small volume compared to the totality of raw data generated through the scanning and sampling operations.

In an embodiment, this information is transferred to a laptop computer for subsequent processing. The overall objective is to identify the nature of the matter under consideration. In particular, when scanning skin conditions, the overall objective is to give an indication as to whether the skin condition is considered benign or whether the skin condition is considered malignant and therefore requires further attention.

As is clear from the data table of *Figure 16*, similar combinations exist for both the calibration stage and the test stage. Thus, specific similar information entries may be directly compared to determine the extent to which they differ. Thus, a larger difference may indicate that skin under test, for example, has characteristics that differ significantly to healthy skin and therefore further investigation should be prompted.

Sophisticated algorithms can be developed for analysing the information generated. For example, extreme values may be removed and weightings may be given for particular electrode combinations. Thus, data derived from lower levels of penetration may be subtracted from data derived from higher levels of penetration to focus on these deeper penetration levels. In addition to or as an alternative, many data sets may be generated and tested by alternative means as a mechanism for training a machine learning system.

### Figure 17

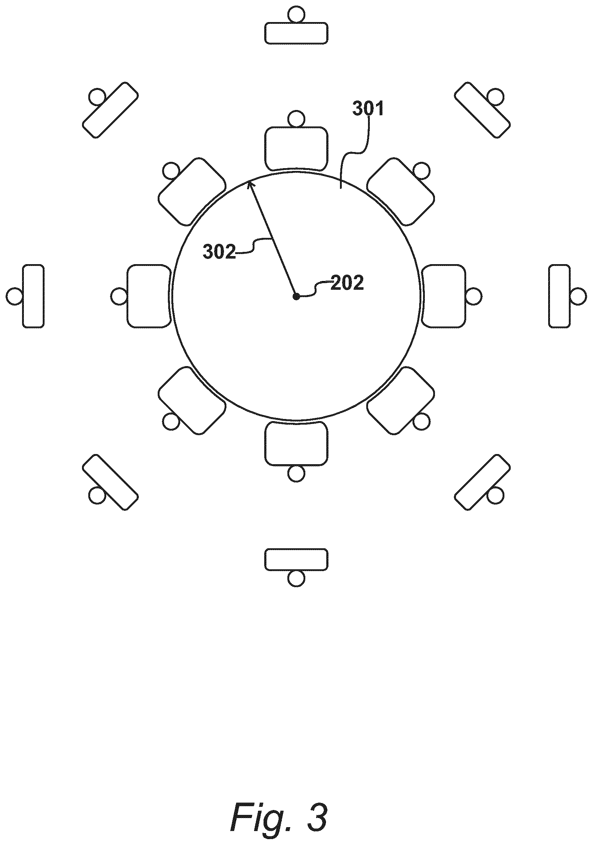

The apparatus described with reference to *Figure 1* to *Figure 16* facilitates the deployment of a method of generating electric fields for penetrating matter to identify one or more properties of this matter. The method may comprise the steps of locating an apparatus such that a hole of a first radius in a dielectric substrate is at the position of the matter for which properties are to be determined. The apparatus is energized to produce electric fields. The dielectric substrate presents a first planar surface and a second planar surface along with a plurality of circumferentially and substantially evenly displaced scanning electrodes which are located on the first planar surface at a second radius around the hole.

Respective electrical conductors for each of the scanning electrodes are configured to pass through the first dielectric substrate to the second planar surface. The apparatus may be deployed within a housing for supporting the dielectric substrate upon a subject's skin and may additionally comprise the step of optically viewing the subject's skin through the hole in the dielectric substrate before initiating the scanning procedures. As described with reference to *Figure 7*, the viewing of the subject's skin may be facilitated by the presence of optical lens **711**, supported within the housing **701.**

The housing **701**, is shown in *Figure 17* located within a cradle **1701.** The cradle **1701** includes a socket **1702** for physically connecting the apparatus, via the cradle, to a laptop computer or similar device. In addition to downloading data (or information as previously defined), the cradle connection to a laptop computer (or similar device) can also be used to upload firmware updates and re-charge the device.

### Figure 18

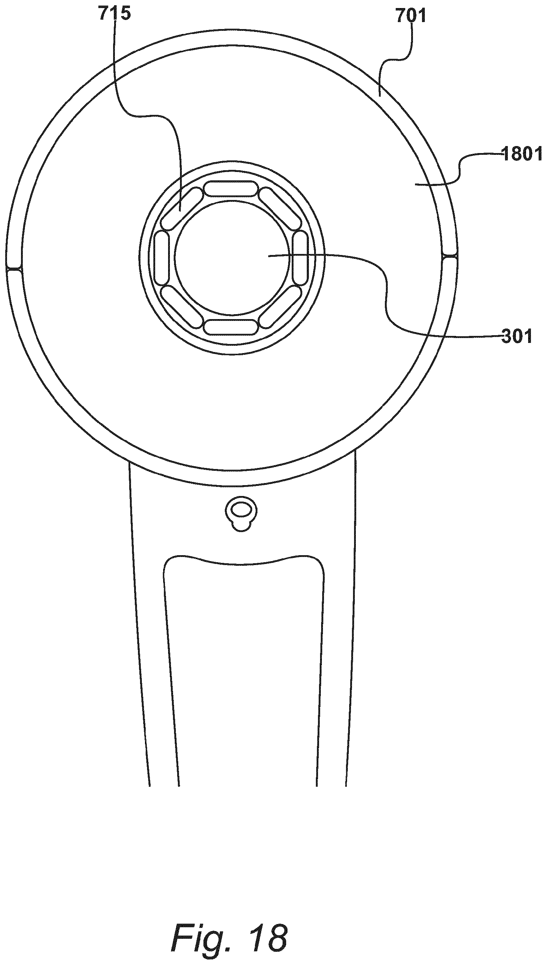

The housing **701** as seen when viewed in the direction of arrow **1703** is shown in *Figure 18*. The housing **701** supports the light emitting devices **715** and these, along with the hole **301** are visible through a transparent portion of a glass cover. The electrodes are obscured by a coloured portion 1801 of the glass cover.

### Figure 19

The arm **1901** of a subject is shown in Figure 19. The skin of arm **1901** includes a region of concern **1902**, possibly in the form of a mole. The light emitting devices **715** facilitate optical viewing of the region of concern by a clinician. Thus, the step of viewing the subject's skin is facilitated by illuminating the skin by means of one or more illuminating devices **715** that are supported within the housing **701.**

### Figure 20

After optically viewing the area of interest **1902**, the apparatus **701** is brought into contact with the subject's skin, as shown in *Figure 20**.* However, the hole **301** is located at the position of interest **1902** such that the electrodes are in contact with the skin, via the glass cover but not at the actual position of interest.

As previously described, the button **713** will have been activated to produce reference data. In the position illustrated in *Figure 20*, button **713** is activated again to perform a scanning cycle to produce test data. As described with reference to *Figure 7*, after the scanning cycle has completed, the colour of light emitting device **714** will change, thereby indicating to an operative that the scanning cycle has completed. The operative may then return the apparatus to the cradle **1701.**

## Claims

1. An apparatus for generating electric fields, wherein said electric fields are suitable for penetrating matter to identify one or more properties of said matter, **characterised by**:
a first dielectric substrate (401) presenting a first planar surface (101) and a second planar surface, with a hole (301) having a first radius;
a plurality of circumferentially and substantially evenly displaced scanning electrodes (111, 112) on said first planar surface, located at a second radius around said hole; and
respective electrical conductors (121, 122) from each said scanning electrode configured to pass through said first dielectric substrate to said second planar surface.

2. The apparatus of claim **1**, **characterised by** a plurality of circumferentially and substantially evenly displaced secondary electrodes (211, 212) on said first planar surface, wherein:
said secondary electrodes are positioned at a third radius;
said third radius is larger than said second radius; and
each said secondary electrode is radially aligned with a respective scanning electrode.

3. The apparatus of claims **1** or claim **2**, **characterised in that**:
said dielectric substrate is implemented as a first circuit board;
said first circuit board is substantially circular and has a fourth radius that is larger than said third radius, comprising a second circuit board (502), wherein:
said second circuit board is substantially parallel with said first circuit board; and
said second circuit board includes electronics for energizing and monitoring said scanning electrodes.

4. The apparatus of claim **3**, wherein said second circuit board includes electronics for introducing a respective resistance between ground and each said secondary electrode.

5. The apparatus of claim **4**, **characterised by** a container (802) for receiving a specimen, wherein said container is locatable upon said first planar surface.

6. The apparatus of claim **4**, **characterised by**:
a tube (901) for receiving a specimen, wherein said tube is locatable within said hole; and
a ring (1001) of dielectric material for surrounding said tube, wherein said ring is configured to concentrate electric fields within the tube, after becoming electrically charged.

7. The apparatus of claim **4**, **characterised by** a housing (701) for supporting the apparatus to facilitate application upon a subject's skin, wherein said housing is configured to support one or more lenses (711) to facilitate optical viewing of the skin through the hole in the dielectric substrate.

8. The apparatus of claim **7**, **characterised by** one or more illuminating devices (715) configured to illuminate an area of skin being optically viewed, wherein said illuminating devices are supported by said housing.

9. A method of generating electric fields for penetrating matter to identify one or more properties of said matter, characterised the steps of:
locating an apparatus such that a hole of a first radius in a dielectric substrate is at the position of said matter; and
energizing the apparatus to produce said electric fields, wherein:
said dielectric substrate presents a first planar surface and a second planar surface;
a plurality of circumferentially and substantially evenly displaced scanning electrodes are located on said first planar surface at a second radius around said hole; and
respective electrical conductors from each said scanning electrode are configured to pass through said first dielectric substrate to said second planar surface.

10. The method of claim **11**, **characterised in that** said energizing step comprises the steps of:
energizing a first scanning electrode;
monitoring an output signal from a capacitively coupled second scanning electrode; and
disconnecting the remaining scanning electrodes, such that the remaining scanning electrodes do not provide a conduction path to ground.

11. The method of claim **10**, **characterised in that** said energizing step comprises the steps of:
selecting each scanning electrode as a first energized electrode; and
for each selected energized scanning electrode, selecting each of the remaining scanning electrodes as a second monitored electrode.

12. The method of any of claims **9** to **11**, **characterised by** the steps of:
energizing a first selected scanning electrode and monitoring a second selected scanning electrode while adjusting electrical characteristics of a secondary electrode radially displaced from the second monitored scanning electrode.

13. The method of claim **12**, **characterised in that** said step of adjusting electrical characteristics includes introducing resistances between ground and each of the secondary electrodes.

14. The method of any of claims **10** to **13**, **characterised by** the steps of:
storing a plurality of monitored output signals; and
analysing said stored output signals to identify a peak value.

15. The method of claim **14**, comprising the step of assessing an interval between the monitored output signal going above a predetermined level and then returning below said predetermined level.
